# EUROPEAN PATENT SPECIFICATION

(11) **EP 2 537 537 B1**
(45) Date of publication and mention of the grant of the patent: **10.12.2014**
(21) Application number: 12185069.7
(22) Date of filing: 26.03.2008
(51) Int. Cl.: A61L 11/00, C02F 1/46, C02F 1/68, C02F 1/467

(54) **Waste treatment system**
Abfallbehandlungssystem
Système de traitement de déchets

(30) Priority: 09.04.2007 US 697921; 09.04.2007 US 697933
(43) Date of publication of application: 26.12.2012
(62) Divisional of application: 08744382.6
(73) Proprietor: Innovation Services, Inc., Knoxville, TN 37922 (US)
(72) Inventor: Dooley, Joseph, Kingston, 37763 (US); Hubrig, Jeffrey, Knoxville, TN Tennessee 37934 (US); Lowden, Richard, Clinton, TN Tennessee 37716 (US)
(74) Representative: Boult Wade Tennant

(56) References cited:
- US-A- 4 880 510
- US-A1- 2002 155 044
- US-B1- 6 274 028

## Description

### TECHNICAL FIELD:

The disclosure relates to an in-line waste and/or modular treatment unit intended to provide for treatment and/or disinfection of liquid infectious wastes, including medical, domestic, scientific, mortuary, or commercial wastes, to disinfect and render the wastes non-infectious and less toxic or less biologically active before flowing the waste stream to a sanitary sewer drain or directly to the environment.

### BACKGROUND AND SUMMARY:

There is growing concern that biological infectious waste streams from hospitals, slaughter houses, and other sources that may contain biologically hazardous or toxic components are not adequately treated before discharging such waste streams to sanitary sewer systems or directly to the environment. Presently, large municipal treatment facilities may not be adequately configured for high concentrations of biological materials originating in hospitals and other sources. Accordingly, there is a need for improved systems and methods for treating waste streams before the streams are discharged into a sanitary sewer system or directly to the environment. There is also a need for modular systems that may be readily deployed as an adjunct to existing sanitary sewer systems at the source of the waste stream, thereby reducing the degree of infectivity of the material a municipal system must treat.

The present invention provides a waste system as set out in claim 1 and a method of treating a waste stream as set out in claim 6. In view of the foregoing and other needs, an exemplary embodiment of the disclosure provides a modular waste disinfection system for substantially liquid infectious waste streams and methods of treating such waste streams. The modular waste disinfection system may include a metal ion generation chamber for introducing selected metal ions into the waste material; an oxidant generation chamber in fluid flow communication with the metal ion generation chamber for disinfection of the waste material with an oxidizing agent; and a chelation chamber in fluid flow communication with the oxidant generation chamber for deactivation of metal ions in the waste material.

Another exemplary embodiment of the disclosure provides a method of treating a sanitary sewer waste material to provide a treated waste stream. The method may include flowing a waste stream from a sanitary sewer drain into a modular waste disinfection system. The modular waste disinfection system may include a metal ion generation chamber for introducing metal ions into the waste material to partially disinfect the waste material; an oxidant generation chamber in fluid flow communication with the metal ion generation chamber for disinfection of the waste material with an oxidizing agent; and a chelation chamber in fluid flow communication with the oxidant generation chamber for deactivation of metal ions and oxidation chemicals in the waste material.

According to the method, the waste stream may be macerated to a predetermined particle size and may be contacted with a film inhibitor and/or a foam inhibitor in the maceration chamber. Metal ions may be generated *in situ* in the metal ion generation chamber for contact with the waste stream from the maceration chamber to disinfect the waste stream. The waste stream may be infused in the oxidant generation chamber by oxidants generated *in situ,* in order to eliminate any biological activity of compounds in the waste stream. The metal ions in the waste stream may then be chelated (bound) in the chelation chamber in order to sequester and deactivate any remaining metal ions and oxidizing chemicals present in the waste stream before discharging the treated waste stream into a sanitary sewer or directly to the environment.

An advantage of the system and methods described herein is that the system combines at least two disinfection techniques in a single unit thereby increasing the effectiveness of waste stream disinfection over the use of a single disinfection technique. Unlike conventional systems, the active disinfection ingredients are deactivated prior to the waste stream being discharged from the disinfection unit so that the disinfection ingredients and waste stream may be discharged to the sanitary sewer system or directly to the environment without removing the disinfection ingredients from the waste stream. Because of the modular nature of the system, the system may be configured as a mobile, or portable, stand-alone unit or may be provided in a substantially fixed non-portable installation that may be inserted between a waste material source and a final disposition of the waste material. The waste treatment system may also be combined and/or integral with a waste collection system or may be configured as a stand-alone system for discharge directly to the environment. Furthermore, portions of the modular system may be employed if the full capability is not required by the nature of the wastes being created.

Another exemplary embodiment provides a modular waste treatment system for liquid waste streams and methods of treating liquid waste streams. The modular waste treatment system includes a maceration chamber for initial treatment and homogenization of waste material; a metal ion infusion chamber in fluid flow communication with the maceration chamber for introducing metal ions into the waste material: and an oxidation chamber for wet oxidation of the waste stream. Provision may be made to recycle the waste to one or more of the chambers for further treatment if required.

Yet another exemplary embodiment provides a method of treating a liquid waste material to provide a treated waste stream. The method may include flowing a waste stream into a modular waste treatment system. The modular waste treatment system may include a maceration chamber for initial treatment and homogenization of waste material; a metal ion infusion chamber in fluid flow communication with the maceration chamber for introducing metal ions into the waste material; and an oxidation chamber for oxidizing oxidizable material in the waste stream. The waste stream is macerated to a predetermined particle size and may be contacted with a film inhibitor in the maceration chamber. Metal ions are generated in the metal ion infusion chamber for contact with the waste stream from the maceration chamber to partially detoxify and to promote the oxidation process of the waste stream. The waste stream is then oxidized in the presence of oxygen to provide a treated stream that is substantially devoid of toxic and active biological materials.

An advantage of the various embodiments described herein is that the system combines at least two disinfection techniques in a single unit thereby increasing the effectiveness of waste stream treatment over the use of a single disinfection technique. The oxidation system used is essentially flameless and therefore does not introduce any combustion products into the atmosphere. However, heating both the waste stream and the oxygen stream may greatly promote the reaction rate. The use of elemental oxygen also provides a much more compact system than units using air (containing only about 20 wt.% oxygen).

Because of the modular components of the system, the system may be configured as a mobile, or portable, stand-alone unit or may be provided in a substantially fixed non-portable installation that may be inserted between a waste material source and a final disposition of the waste material. The waste treatment system may also be combined and/or integral with a waste collection system. In another embodiment, the waste treatment system may be a stand alone system for discharge of treated wastes to the environment.

Additional objects and advantages of the disclosure are set forth in part in the description which follows, and/or may be learned by practice of the disclosure. The objects and advantages of the disclosure may also be realized and attained by means of the elements and combinations particularly pointed out in the appended claims.

It is to be understood that both the foregoing general description and the following detailed description are exemplary and explanatory only and are not restrictive of the disclosure, as claimed.

### BRIEF DESCRIPTION OF THE DRAWINGS:

Further advantages of the exemplary embodiments may become apparent by reference to the detailed description of the exemplary embodiments when considered in conjunction with the following drawings illustrating one or more non-limiting aspects thereof, wherein like reference characters designate like or similar elements throughout the several drawings as follows:
FIG. 1 is a block flow diagram of one embodiment of a method of the present disclosure;
FIG. 2 is a schematic representation of one non-limiting example of a substantially linear waste disinfection unit according to a preferred embodiment;
FIG. 3 is a perspective, cut-away view, not to scale, of a substantially linear waste disinfection unit according to an embodiment of the disclosure;
FIG. 4 is a perspective, cut-away view, not to scale, of a substantially vertical waste disinfection unit according to another embodiment of the disclosure;
FIG. 5 is a block flow diagram of a treatment process according to the disclosure;
FIG. 6 is an overall schematic drawing of a modular flameless waste treatment system according to the disclosure;
FIG. 7 is an enlarged schematic drawing of a portion of the treatment system of FIG. 6 including a receiving chamber and a metal ion infusion chamber; and
FIG. 8 is an enlarged schematic drawing of a portion of the treatment system of FIG. 6 including an oxidation chamber.

### DESCRIPTION OF THE EXEMPLARY EMBODIMENTS

### In-Line Waste Disinfection Llnit Embodiment

As described in more detail below, embodiments of the present disclosure may provide systems and methods for disinfecting substantially liquid infectious waste streams before discharging the waste streams to a sanitary sewer system or directly to the environment. The deactivation or destruction of infectious agents, such as viruses, bacteria, protists, fungi, algae, prions, or other infectious organic matter, through embodiments of the present disclosure may be herein referred to as "disinfection" or "biocidal activity." The systems and methods may be adaptable to being portable or to being permanently attached to existing sanitary sewer drains. Each system may be substantially self-contained so that fluid discharged from the system may be suitable to flow into an existing sanitary sewer or directly to the environment without further disinfection.

The systems and methods of the present disclosure may generate reactive disinfection agents *in situ* during the course of operation. Waste streams may be treated with a synergistic combination of metal ions and oxidants, such as hypochlorites, peroxides, or hydroxyl ions. However, it is not desirable to discharge metal ions and oxidants into the sewer system. Therefore, the waste streams may be further treated to ensure deactivation of the reactive agents and allow for discharge of the waste stream through a sewer system or directly to the environment.

The oxidants may be generated electrolytically *in situ* from water having minimal amounts of common salt (sodium chloride) present or from salts existing in organic tissues that may be present in the waste. The metal ions may likewise be generated *in situ* via redox reactions when electrodes comprising the appropriate metals are subjected to an electrical current of suitable polarity, voltage, and time duration. The ability to generate both metallic ions and reactive nonmetallic compounds *in situ* by electrolytic redox reactions provides a key disinfecting technique, generating the biocidal species on the spot as they are needed. Any active disinfecting agents not chemically deactivated as a result of the disinfection process may then be bound and deactivated by subsequent processing at the end of the disinfection cycle.

Unlike conventional disinfection systems, the system described herein may bind and deactivate the metal ions used for disinfection before discharge of a treated fluid stream to the sewer system, rather than removing the metal ions by precipitation or other reaction mechanism. The presently disclosed system may charge a sufficient quantity of a chelating agent, such as EDTA or citric acid, to a chelation chamber, which may bind the metal ions and inactivate them. Chelation may also serve to bind unreacted oxidants that may be present in the stream. This binding technique is similar to the treatment used by physicians to treat ingestion of toxic metals for excretion from the body, and may allow for the chelated metals to be safely discharged into the sewer system or directly to the environment.

The present disclosure may also exploit the powerful biocidal properties of oxidizing agents, such as hypochlorites and peroxides. The aforementioned and related chemicals, generally known as oxidants, are well known and established as highly effective microbicides. Oxidation also serves to break down organic molecules, such as pharmaceuticals, into less objectionable compounds.

Hypochlorites may also display effectiveness against viruses by virtue of an ability to attack and denature proteins. This feature makes them effective against viruses which may be either coated with an external protein coat, or uncoated. The oxidizing agents made and used in the system for their biocidal activity may also be deactivated or neutralized in situ by spontaneous reactions with organic compounds in the waste, without the need to remove these chemicals from the treated fluid stream.

While both metal ions and oxidizing agents are known to be effective biocidal agents individually, the combination of metal ions and oxidants in a single system may provide a synergislically improved effectiveness in biocidal activity that may be about 100,000 times greater than the disinfectant effectiveness of either the metal ions or the oxidants alone.

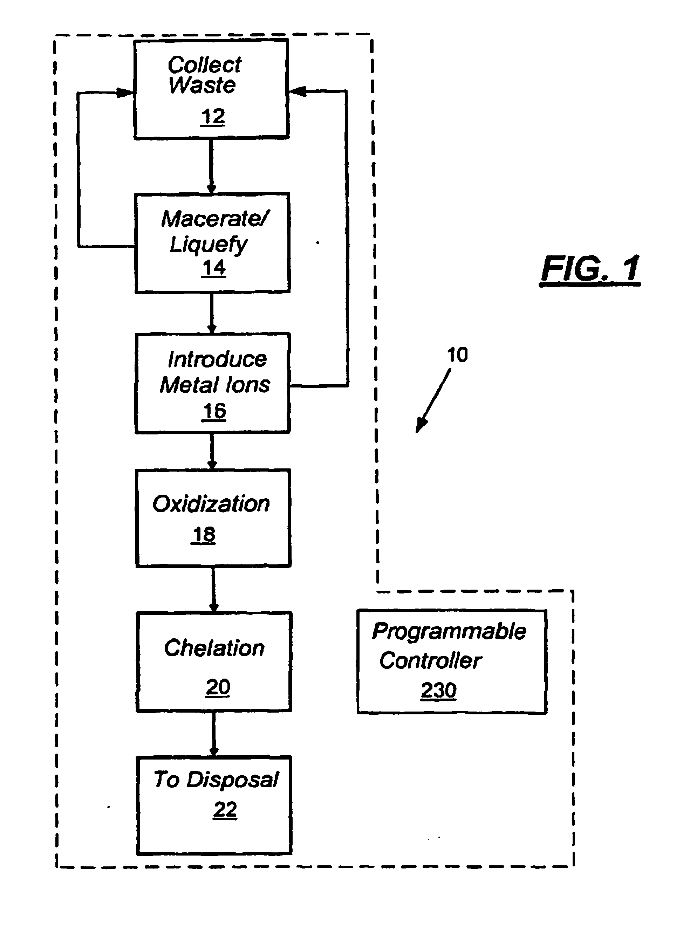

With reference to FIG. 1, one embodiment of a method of the present disclosure provides a system 10 that includes a series of continuous, in-line processes for the disinfection of a substantially liquid infectious waste stream, with each process stage further described in more detail below. Each process stage may herein be represented as an individual physical chamber in order to provide a clearer understanding of the in-line process concept. However, the present disclosure is not limited to individual chambers for each process stage, as in an alternative embodiment discussed below. The presently described process stages may be implemented either in individual chambers to simplify control or be combined in common chambers to achieve an optimum footprint for the physical dimensions and cost/benefit to the unit design configuration.

Infectious waste is first collected in a waste collection step 12, where water may be added if necessary to ensure that the waste material is substantially liquid or in substantially liquid form for further treatment. Accordingly, the waste material may also be macerated in a maceration step 14. The maceration step 14 may ensure a homogenous particulate size for any organic matter that may be present in the substantially liquid waste material. The term "substantially liquid" means that any solids present in the waste material remain substantially suspended in a liquid phase for flow through the system 10.

If the particulate size of any organic matter present is too large for the waste to be processed in the next process step, the waste material may be recycled for further maceration in the maceration step 14. Next, metal ions may be introduced into the waste material in a metal ion generation step 16, where electrolysis of a sacrificial electrode may generate oligodynamic concentrations of metal ions *in situ.* The waste material may remain at or be recirculated through this stage for a period of several minutes, in order to infuse an adequate concentration of metal ions into the waste material and/or to allow sufficient time for the metal ions to at least partially disinfect the waste material.

In the next step of the process, the waste material may be oxidized in an oxidation step 18, wherein reactive oxidizing ions, for example hypochlorite, may be electrolytically generated *in situ.* The waste material may be treated with oxidizing ions for a period of several minutes, in order to allow sufficient time to produce adequate concentrations of oxidizing ions in the waste material and/or for the oxidants to at least partially disinfect the waste material. There may be residual metal ions from the metal ion generation step 16 present during the oxidation step 18.

After the oxidation step 18, the waste material may then be passed to a chelation step 20, wherein any metal ions remaining in the waste material may be bound to a chelating agent, thereby removing such ions from the treated waste material. The waste material may remain at this stage for a period of several minutes, in order to allow sufficient time for the chelating agent to sequester the metal ions. Chelation may also serve to bind unreacted oxidants that may be present in the stream. Finally, the waste material may be discharged or disposed of through a waste disposal step 22 to a sanitary sewer system or directly to the environment.

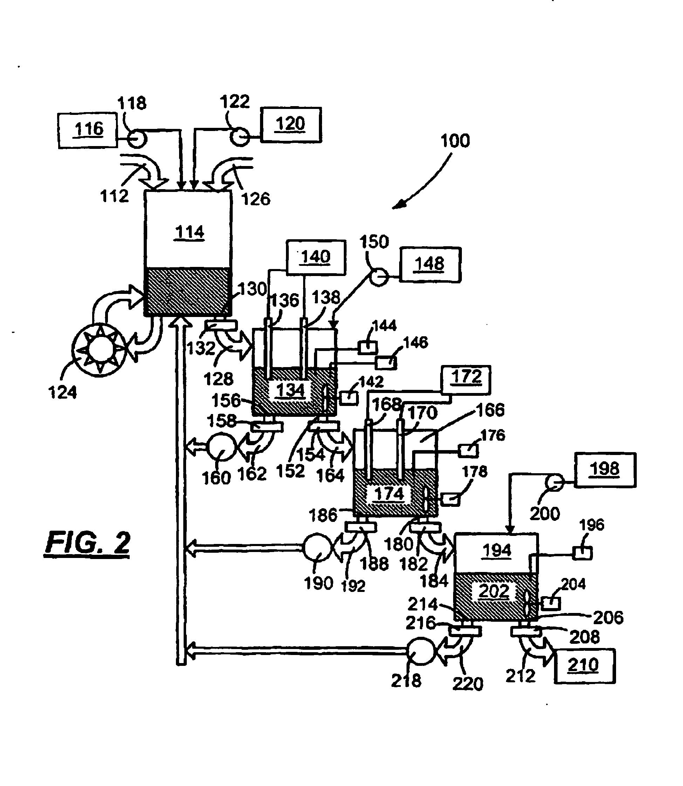

In order to further illustrate aspects of exemplary embodiments of the disclosure, reference is now made to FIG. 2. FIG. 2 is a non-limiting, schematic illustration of a modular system 100 including the process steps described above with reference to FIG. 1.

As shown in FIG. 2, a substantially liquid infectious waste stream 112 comprising biological waste may be fed into a holding chamber 114. A film inhibitor reservoir 116 including a film inhibitor dosing pump 118, and a foam suppressant reservoir 120 including a foam suppressant dosing pump 122 may be associated with the holding chamber 114 for feeding a film inhibitor and/or a foam suppressant into the holding chamber. A macerator 124 may also be associated with the holding chamber 114, wherein the waste material in the holding chamber 114 may be mechanically macerated to reduce the size of any solid material in the holding chamber and to combine the waste with water or other aqueous solution that may be introduced to the holding chamber 114, as needed, via an additional input line 126. The holding chamber 114 may be constructed of copper or copper alloy to provide an inherent bactericidal action thereby suppressing undersirable bacterial growth. Similar bactericidal action may be obtained by copper plating or use of a copper or copper alloy floorplate in the holding chamber 114.

The metered dose of a film inhibitor, such as sodium lauryl sulfate ("SLS") is believed to perform two critical functions. First, the film inhibitor may initiate a chemical attack to begin breaking down and denaturing both lipid and protein complexes present in the waste. Second, the film inhibitor's inherent detergency properties may enable the holding chamber 114 to remain "self-cleaning." As an additional feature, SLS is also well known as a disinfecting agent, and it may contribute to the overall synergistic disinfectant effect of the present system 100. An amount of SLS that may be metered into the holding chamber 114 may range from about 0.1 to about 10.0 percent by volume.

The foam suppressant, such as a silicone-based antifoam agent, may further ensure that air bubble formation is reduced during maceration cycles, so that air entrapment does not inhibit the operating efficacy of the reactive disinfectant species generated in the subsequent disinfection chambers. An amount of foam suppressant that may be used to suppress air entrapment in the holding chamber 114 may range from about 0.05 to about 1.0 percent by volume.

The holding chamber 114 may employ one or more macerators 124 for chopping or mixing material within the chamber 114 in order to macerate and mix the incoming waste stream 112 with the water and the solution of film inhibitor and foam suppressant. Macerating the waste stream 112 may also extend the time of contact between the waste stream 112 and metal ions or oxidizing chemicals in the system 100. The initial maceration may aid to break down organic solids in the waste stream 112 to a homogenized rough particle size and may introduce water or other aqueous solution carrier necessary to establish a waste stream 128 flow into a subsequent chamber.

A suitable particle size exiting the holding chamber 114 may be less than about .5 millimeters in diameter and typically less than .3 millimeters in diameter after maceration. For example, the maximum particle size exiting from the macerator 124 may range from about 0.25 to about 0.5 millimeters in diameter. The initial particle size of particles entering the macerator may range from about 5 to about 10 millimeters in diameter. The term "diameter" is used to signify an average cross-sectional dimension of particles based on the largest cross-section of the particles in the waste stream 112 and is not intended to indicate that the particles are necessarily circular or spherical.

The holding chamber 114 may further comprise a fluid exit port 130 comprising a unidirectional valve 132 and/or a pump for allowing a sufficiently homogenized waste stream 128 to enter a metal ion generation chamber 134 that may be in fluid flow communication with the holding chamber 114.

The metal ion generation chamber 134 contains at least one pair of electrodes 136, 138 that may be electrically connected to a power supply 140. The electrodes 136, 138 may comprise one or more metals, including aluminum, silver, copper, bismuth, gold, or zinc. The metal composition of the electrodes 136, 138 may provide a source for the electrolytic generation of corresponding metal ions.

The power supply 140 may provide electrical energy for the electrodes 136, 138. Application of electrical energy to the electrodes 136, 138 may cause metal ions to be liberated from the electrodes via one or more redox reactions. The liberated metal ions may then become dissolved in the waste mixture particulate suspension in the metal ion chamber 134 so that the ions may provide disinfecting activity to the waste. The voltage and current applied to the electrodes 136, 138 may be externally regulated in order to exercise control over the concentration of metal ions that may be dissolved in the waste suspension in the chamber 134.

Multiple pairs of electrodes 136, 138, each electrode comprising one or more metal compositions and having an appropriate voltage and current flow, may be used to introduce various concentrations of one or more metal ions into the waste mixture suspension. The metal ion generation chamber 134 may itself be used as one of the electrodes.

The dissolved metal ions may act oligodynamically within the waste suspension to deactivate or destroy bacterial, protist, fungal, algal, prion, and viral infectious agents present within the waste. It is believed that a total metal ion concentration of at least several parts per million is suitable for disinfection purposes.

In one embodiment, both silver and copper ions may be produced. It is believed that a concentration of copper ions that is much greater than a concentration of silver ions is particularly suitable for disinfection of waste liquids. Although a concentration ratio of 10:1 Cu to Ag has been found to be highly effective, other ratios may prove to be suitable for this application. Accordingly, ions of different metals may be produced at different concentration levels in order to provide a suitable total dissolved metal ion concentration. In one embodiment, a suitable copper ion concentration may range from about 100 ppm to about 1000 ppm, with a further suitable example being about 400 ppm of copper ions. Likewise, suitable silver ion concentration may range from about 10 ppm to about 100 ppm, with a further suitable example being about 40 ppm of silver ions. A suitable total metal ion concentration for disinfection may range from about 110 ppm to about 1100 ppm. As a further example, a suitable total metal ion concentration may range from about 200 ppm to about 800 ppm, and as another suitable example a total metal ion concentration may range from about 300 ppm to about 600 ppm.

A metal ion exposure time ranging from about 1 to about 30 minutes may be suitable to provide disinfection to the waste stream, with a further suitable example ranging from about 5 to about 10 minutes of exposure time. Particularly resistant wastes may require additional time or higher concentration of the metal ions. Variations in operation may be accommodated by process control using a programmable controller as part of the system 100.

The electrodes 136, 138 used to produce the metal ions may be pure metals in which multiple pairs of electrodes 136, 138 may be used and voltages and currents to each electrode 136, 138 pair regulated independently in order to control the various metal ion concentrations. The electrodes 136, 138 may also be composed of a mixture of more than one metal, such as a metal alloy, in order to control the concentration of each ion in solution. Each of the electrodes in the pair of electrodes 136, 138 may comprise a distinct and independent composition.

The electrodes 136, 138 may be fabricated employing powder metallurgy. A further embodiment using copper powder and silver or silver-alloy "solder" may be employed as a binder. The powdered metal electrodes 136, 138 may be fabricated such that the concentration of exposed metals such as copper or silver could be carefully controlled to produce the desired concentration ratio of metal ions. Additionally, the composition of each electrode 136 or 138 and its corresponding ionic contribution may be controlled through particle size and amount of each phase, primary metal and "binder" present in the powder molded electrode 136 or 138. For example, large spherical grains of copper may be pressed with silver solder powder and sintered to form an electrode 136 with higher surface concentrations of copper. Studies have shown that a combination of copper and silver ions wherein the concentration of copper ions is much higher than the concentration of silver ions may be very effective in disinfecting liquid containing biological hazards.

In a further embodiment of the present disclosure, one or more electrodes 136, 138 may be integrated into a mixing device or mixing pump 142 in which the various vanes or other portions of the mixing device may also act as an electrode. In an alternative embodiment, a mixing device 142 may comprise an electrode and may be used in combination with electrodes 136, 138.

The metal ion generation chamber 134 may further comprise a fluid level meter 144, a conductivity meter 146, a saline reservoir 148 and a saline dosing pump 150. The metal ion generation chamber 134 may also comprise a fluid exit port 152 comprising a directionally restrictive fluid flow valve 154 and/or pump. Hence, the metal ion generation chamber 134 may be in fluid flow communication with at least one subsequent treatment chamber. A second fluid exit port 156 comprising a recycling valve 158 and/or pump 160 may allow at least a portion 162 of the waste treated in the metal ion generation chamber 134 to be returned to the holding chamber 114 so that the portion 162 of waste may be recycled through the system 100 and further disinfected.

The metal ion treated waste stream 164 may then be passed into an oxidant generation chamber 166. The oxidant generation chamber 166 may comprise a set of electrodes 168, 170, each composed of a material selected from carbon, titanium, stainless steel, and relatively inert materials.. The electrodes 168, 170 are in electrical connection with a power supply 172. Application of electric current to the electrodes may cause hypochlorite or other reactive, disinfectant, oxidant species to be generated and to flow through, and dissolve in, the waste mixture particulate suspension 174 present within the oxidant generation chamber 166.

A suitable oxidant concentration may range from about 0.10 ppm to about 10 ppm, with a further example of a suitable oxidant concentration ranging from about 1 ppm to about 5 ppm. An oxidant exposure time ranging from about 1 to about 20 minutes may be suitable to provide disinfection to the waste suspension 174, with a further suitable example being from about 5 to about 10 minutes of exposure time. Particularly resistant wastes may require additional time or higher concentration of the metal ions. Variations in the oxidant concentration be accommodated by process control using a programmable controller.

The oxidant generation chamber 166 may also comprise a fluid level sensor 176 and a mixer 178. A fluid exit port 180 comprising a directionally restrictive fluid flow valve 182 and/or pump may allow oxidant treated waste 184 to be passed to a subsequent chamber in fluid flow communication with the oxidant generation chamber 166. A second fluid exit port 186 comprising a recycling valve 188 and/or pump 190 may allow at least a portion 192 of the waste suspension 174 to be passed to the holding chamber 114 or to a previous chamber so that the portion 192 of waste suspension 174 may be recycled through the system 100 and further disinfected.

The oxidant treated waste 184 may then be passed into a chelation chamber 194 comprising a fluid level sensor 196, a chelating agent reservoir 198, and a chelating agent dosing pump 200. A quantity of chelating agent may be provided to the waste material 202 in the chelation chamber 194 from the reservoir 198 by the dosing pump 200 in order to facilitate removal of the metal ions. A mixing device 204 may provide continuous circulation and contact of the waste 202 with the chelation agent in the chelating chamber 194. The waste 202 may be processed in a timed chelation cycle that may allow the metal ions to be bound chemically to the chelating agent and may ensure that the oxidants have fully reacted with any organic materials present in the suspension.

The timed chelation cycle may range in duration from about 1 to about 30 minutes, with another suitable example being from about 5 to about 10 minutes. The amount of chelating agent in the chelation chamber 194 sufficient to chemically bind any metal ions may range from about a molar equivalent of the metal ion concentration to about one and a half times a molar equivalent of the metal ion concentration. Typically, the amount of chelating agent will be about a molar equivalent of the metal ion concentration in the waste 202.

A suitable chelating agent may be selected from EDTA, citric acid, sodium citrate, acetylacetone, ethylenediamine, diethylenetriamine, tetramethylethylenediamine, 1,2-ethanediol, 2,3-dimercaptopiopanol, porphyrin, gluconic acid, or similar compounds.

Following completion of the timed chelation cycle, the treated waste 202 may be discharged through a fluid exit port 206, comprising a unidirectional valve 208 and/or pump, into a sanitary sewer system 210 in a disinfected and chemically inert state by either pumping or gravity flow into a sanitary sewer drain. A sewer discharge connection 212 may serve to maintain fluid flow communication between the disinfection system and the sewer system. The chelation chamber 194 may further comprise a second fluid exit port 214 comprising a recycling valve 216 and/or pump 218 that may allow at least a portion 220 of the waste material 202 to be returned to the holding chamber 114 or a previous chamber so that the portion of waste 220 may be recycled through the system 100 and further disinfected.

Embodiments of the present disclosure may also comprise a programmable controller 230 (FIG. 1) capable of interfacing with the fluid level sensors 144, 176 and 196, conductivity sensor 146, and other sensors that may be present in order to coordinate the activities of dosing pumps 118, 122, 150 and 200, valves 132, 154, 158, 182, 188, 208 and 216, pumps 160, 190, and 218 and macerator124, to estimate the amount of waste being processed, to control the electrode voltage and currents responsible for producing the active disinfecting agents, and to control the time intervals for each stage of waste processing. The controller 230 may also estimate the amount of chelating agent required in the chelation chamber based on sensor feedback.

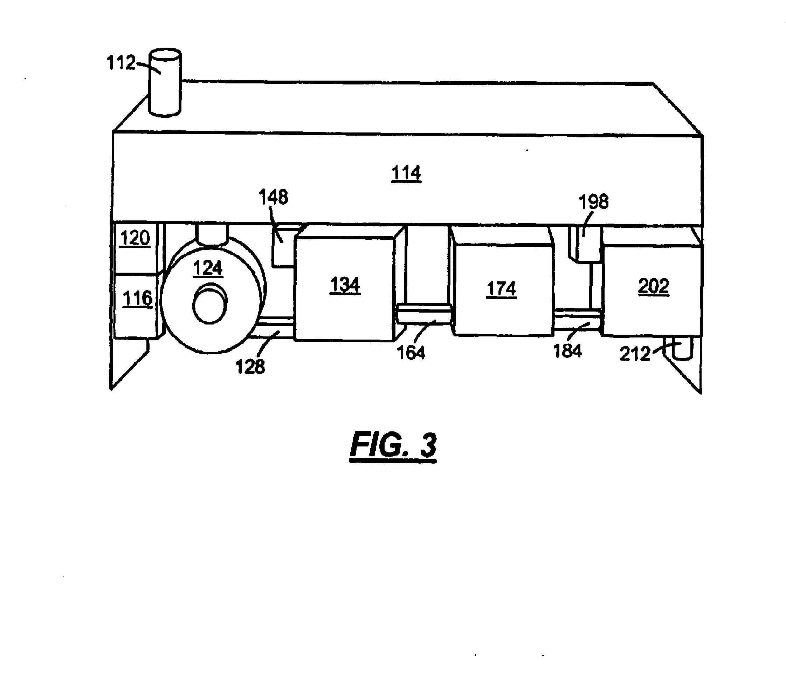

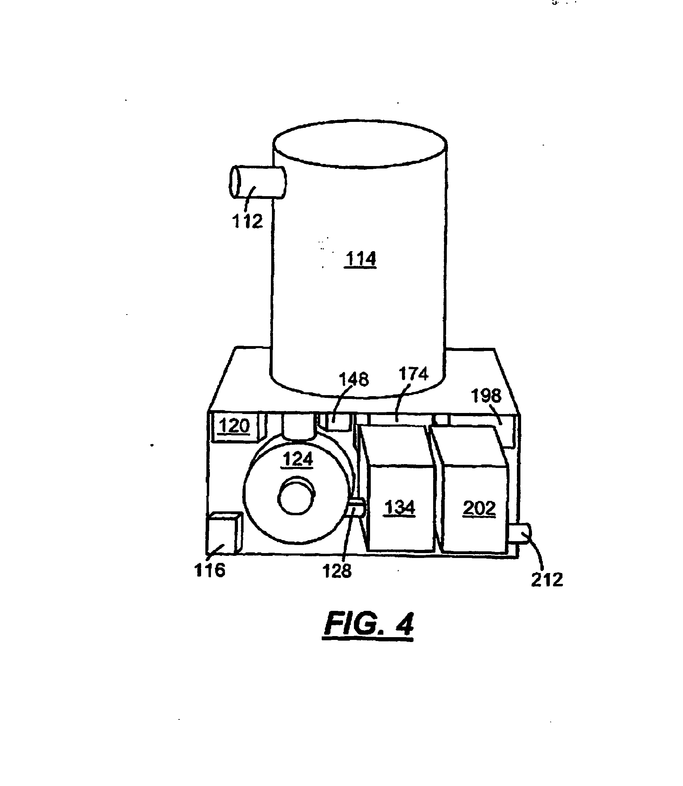

Compact arrangements of the components of the system 100 are illustrated, for example in FIGS. 3 and 4. FIG. 3 is a substantially linear arrangement of the components of the system 100 described above. In the linear arrangement depicted in FIG. 3, the overall height of the system 100 is minimized so that the system may be installed under an existing sink. Accordingly, the dimensions of such a system may range from about 61cm (24 inches) to about 91cm (36 inches) in length, from about 30cm (12 inches) to about 41cm (16 inches) in width, and from about 38cm (15 inches) to about 51cm (20 inches) in height.

An alternative arrangement of the components of the system is illustrated in FIG. 4. FIG. 4 depicts a substantially vertical arrangement of the components of the system. The system depicted in FIG. 4 may have overall dimensions ranging from about 61cm (24 inches) to about 76cm (30 inches) square, and from about 61cm (24 inches) to about 91cm (6 inches) in height. Other arrangements of the components of the system may be possible so that a reduced height and a reduced length are provided. However, it is desirable that the components be arranged in a compact manner so that the system 100 is relatively compact and/or portable.

The system 100 may be particularly adapted to treating waste liquid streams 112 containing bacteria, surgical waste, biological or biologically toxic materials. Such materials may include, but are not limited to, dairy shed waste, fowl waste, milk processing plant waste, food processing wastes, waste from the wine and brewery industries, food waste, shipboard waste, sewage, medical waste, and the like.

In a further embodiment, an ability to reverse the direction of electrical current flow between a pair of electrodes may be desirable to prevent and remove a build-up of slime-like coatings of organic materials or a buildup of residual mineral scale which may form as a by-product of the electrolytic reactions used to generate the disinfecting agents. Such coatings or scale may impede the generation of further disinfecting agents over time. The ability to remove mineral scale or coatings by a reversal of electrical current flow may allow the electrodes to have a longer effective disinfectant generation lifetime, and may provide an economic benefit by decreasing the frequency of electrode replacement.

In embodiments of the present disclosure, replaceable electrodes and replaceable cartridges or refillable reservoirs of chelating agents, film inhibitors, foam suppressants, and saline solution may be provided. In one embodiment, the cartridges or reservoirs and the electrodes are accessible from the exterior of the system in order to facilitate ease of refilling or replacement by the user.

The individual process stages, discussed above as occurring in a separate chamber for each stage, are not limited to such an embodiment. Multiple stages may occur concurrently in a single physical chamber. For example, in an alternative embodiment, the metal ion generation chamber 134 and the oxidant generation chamber 174 may be combined into a single electrochemical disinfection chamber, where the simultaneous generation of metal ions and oxidizing agents *in situ* may provide a greater synergistic disinfectant activity, and a more efficient cost-effective disinfection process.

A trimming electrode (not shown) may be added to the combined chamber in order to ensure that the current level may be adjusted to provide for suitable concentration levels of the active metal ions and oxidants. The trimming electrode may be in electrical communication with the controller 230 and power supply.

### Modular Flameless Waste Treatment Unit Embodiment

As described in more detail below, some embodiments of the disclosure provide further systems and methods for treating waste streams before discharging the waste streams to a sanitary sewer system or directly to the environment. The systems and methods may be adaptable to being portable or being attached to existing sanitary sewer drains for multiple locations. Each system may be substantially self-contained so that fluid discharged from the system may be suitable to flow into an existing sanitary sewer without further treatment or for flow directly to the environment without further treatment.

The systems and methods of the present disclosure may generate reactive disinfection agents in situ during the course of operation of the system. Waste streams may be treated with a synergistic combination of metal ions and a wet oxidation step. Because the metal ions are effective at low concentrations and because they are rendered inert during oxidation; no further treatment to remove the metal ions is required. An oxygen demand sensor may be included to ensure the effectiveness of treatment before discharge of the treated stream to a sewer system or the environment.

While metal ions used in combination with oxidizing agents such as chlorine are known to be effective biocidal agents, the combination of metal ions and a wet oxidation step in a single system may provide a synergistically improved effectiveness for eliminating biological activity and reducing the toxic effect of components in the waste stream.

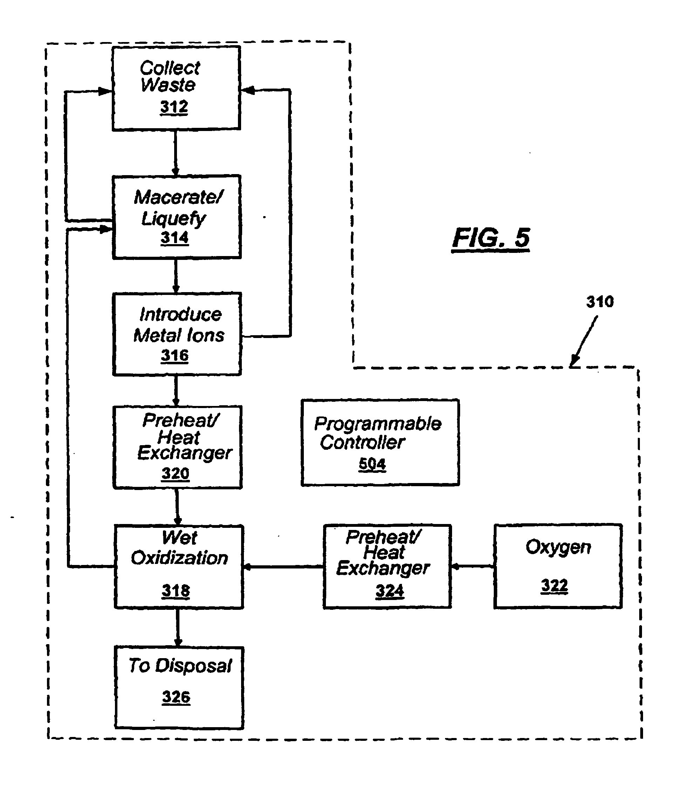

With reference to FIG. 5, an embodiment of the disclosure provides a waste treatment unit 310 for performing a series of continuous, in-line processes, with each process stage described in more detail below. Each process stage may herein be represented as an individual physical chamber in order to provide clear understanding of the in-line process concept.

As illustrated schematically in FIG. 5, an overview of the treatment system 310 is presented. The treatment system 310 includes a waste collection step 312 for collecting waste liquid to be treated. After collection, the waste liquid is macerated in a maceration step 314 in order to reduce the size of solid particles and homogenize the particles and waste in the waste stream to a size that can be effectively treated with metal ions and wet oxidation in later stages of the process. As shown, the macerated liquid may be recycled to mix with the waste liquid being collected in step 312 to promote mixing and homogenization and to extend the time of contact with the active chemicals. Water or other aqueous solution may be added to the waste collected in step 312 to promote flow through the system 310.

Next, a metal ion infusion step 316 is provided to generate or otherwise infuse metal ions into the waste liquid for metal ion treatment of the waste liquid and to promote the oxidation process. The macerated liquid may be recirculated through the metal ion infusion stage in multiple passes to ensure adequate concentrations of metal ions.

The metal ion treated liquid is then oxidized in a wet oxidation step 318. The wet oxidation step may be enhanced by heating the metal ion treated liquid in a preheat step 320 prior to the wet oxidation step 318. The wet oxidation step 318 is conducted using oxygen gas 322, in conjunction with the metal ions to effect a low temperature chemical reaction. According to embodiments of the disclosure, pure oxygen is the most desirable oxygen gas for conducting the oxidation step of the process 310 because it substantially reduces the volume requirements for treating the waste. Based upon the readings from an oxygen demand sensor, the waste may be recycled or may be suitable for disposal according to step 326 of the process 310.

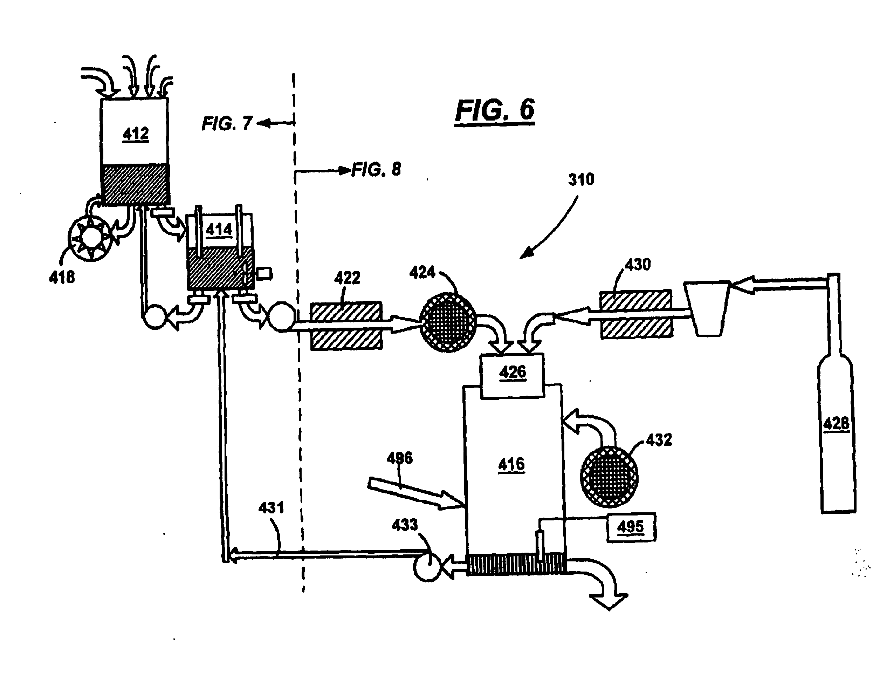

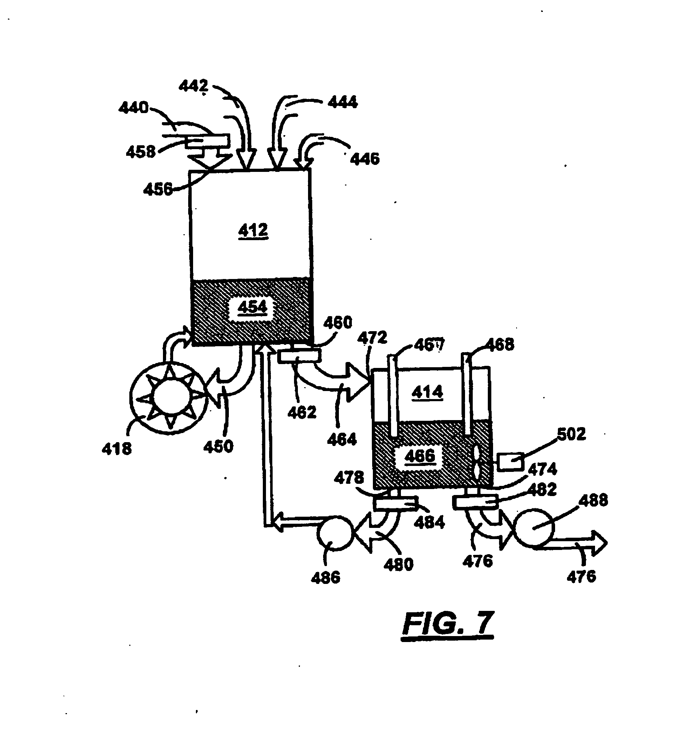

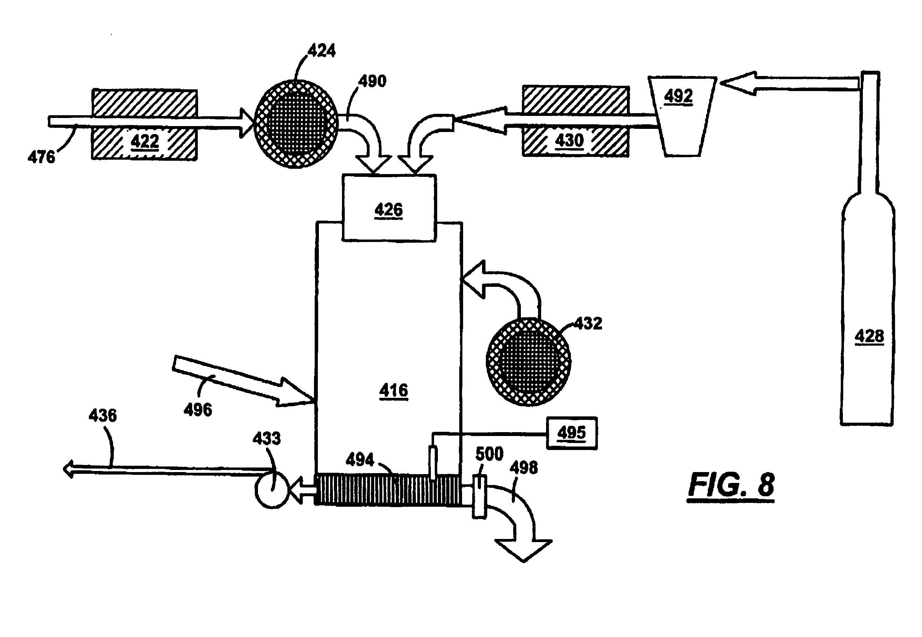

As shown in FIG. 6, the main components of the system 310 include a receiving chamber 412, a metal ion infusion chamber 414, and an oxidation chamber 416. A macerator 418 may be associated with the receiving chamber 412 or may be associated with the metal ion infusion chamber 414 or both. Additional process chemicals such as an anti-foam and a film inhibitor may be provided to the waste liquid in the receiving chamber 412. A heat exchanger or other heating means 422 is provided to preheat the metal ion treated liquid prior to introducing the treated liquid into the oxidation chamber. The metal ion treated liquid is also pressurized with a pressure pump 424 for feeding into a spray mixing nozzle 426 for feed, along with an oxidizing agent, into the oxidation chamber 416. An oxygen source 428 is provided for feeding oxygen gas to the oxidation chamber 416. The oxygen gas may also be preheated in a heat exchanger or by other heating means 430. The oxidation chamber 416 may also be pressurized by a compressor or pressure pump 432 associated with the chamber 416. A portion of the metal ion treated and oxidized material in the oxidation chamber 416 may be recirculated through recirculation line 431 by recirculation pump 433 to the metal ion infusion chamber 414. Further details of the components of the system 310 are provided in FIGS. 7 and 8 discussed below.

### Deceiving Chamber

As shown in FIG. 7, a waste stream 440 comprising biological waste may be fed into the receiving chamber 412 where it may be mechanically macerated in the macerator 418 and combined with tap water 442 or other aqueous fluids such as a saline solution to provide a pumpable slurry. Depending on the biological or lipid content of the waste stream, a metered dose of a lipid/protein complex film inhibitor 444 may be provided to the receiving chamber 412. A suitable film inhibitor 444 may be an aqueous solution of sodium lauryl sulfate (SLS). Optionally, a foam suppressant 446, such as an organosilicone compound, may also be added to the receiving chamber 412 to reduce foaming tendencies of the waste stream 440. In an alternative embodiment, metal ions from a metal ion solution may be metered into the receiving chamber using a dosing pump. The receiving chamber 412 may be constructed of copper or copper alloy to provide an inherent bactericidal action thereby suppressing undesirable bacterial growth. Similar bactericidal action may be obtained by copper plating or use of a copper or copper alloy floor plate in the receiving chamber 412.

The film inhibitor 444 is believed to perform two critical functions. First, the film inhibitor 444 may initiate a chemical attack to begin breaking down and denaturing both lipid and protein complexes present in the waste material 454. Second, the film inhibitor's inherent detergency may enable the receiving chamber 412 to remain "self-cleaning". An amount of film inhibitor 444 that may be used in the receiving chamber 412 may range from about 0.1to about 10 percent by volume.

The foam suppressant 446 may further ensure that air bubble formation in the waste material 454 is reduced during maceration cycles, so that air entrapment does not inhibit the operating efficacy of subsequent treatment chambers. An amount of foam suppressant 446 that may be used in the receiving chamber 412 to suppress air bubble formation may range from about 0.05 to about 1.0 percent by volume.

The receiving chamber 412 may include a macerating device 418, such as a pump, a rotary paddle or blade, or other means of chopping or mixing the recirculated stream 450 from the chamber 412 in order to macerate and mix the incoming waste stream 440 with the water 442, film inhibitor 444 and/or foam suppressant 446. Maceration may effectively break down waste solids in the waste material 454 to a common rough particle size and to suitably mix waste solids in the waste material 454 with sufficient fluid for flow to subsequent chambers for waste processing. Multiple maceration steps may be used to provide a suitable particle size in the waste material 454 for subsequent treatment.

Accordingly, the waste material 454 is macerated to provide a substantially liquid stream. The term "substantially liquid" means that any solids present in the waste material remain substantially suspended in a liquid phase for flow through the system 310.

A suitable particle size exiting the receiving chamber 412 may be less than about .5 millimeters in diameter and typically less than .3 millimeters in diameter after maceration. For example, the maximum particle size exiting from the macerating device 418 may range from about 0.25 to about 0.5 millimeters in diameter. The initial particle size of particles entering the macerating device 418 may range from about 5 to about 10 millimeters in diameter. The term "diameter" is used to signify an average cross-sectional dimension of particles based on the largest cross-section of the particles in the waste material 454 and is not intended to indicate that the particles are necessarily circular or spherical.

The chamber 412 may further include a fluid inlet port 456 that may include a unidirectional inlet valve 458 or pump unit for allowing the waste stream 440 to enter the chamber 412. The chamber 412 may have a fluid exit port 460 containing a unidirectional exit valve 462 or pump unit for allowing the waste stream 464 as a slurry or suspension of solids to exit the chamber 412. The inlet and exit ports 456 and 460 may be positioned on opposite sides of the receiving chamber 412, or they may be otherwise configured to provide flow into and out of the chamber 412 as required to maintain a predetermined level of liquid in the chamber 412. In that regard a suitable level control device may be used to maintain a predetermined level of fluid in the chamber 412.

### Metal Ion Infusion Chamber

The waste stream 464 may then flow from the receiving chamber 412 into the metal ion infusion chamber 414 where a set of electrodes comprising an anode 467 and a cathode 468, each composed of one or more metals selected from silver, copper, zinc, bismuth, gold, aluminum and/or other metals may be immersed in the waste material suspension 466. Application of electrical energy to the anode 467 and the cathode 468 may cause metal ions to be liberated from the electrodes via one or more redox reactions, whereby the metal ions may become dissolved in the waste material suspension 466. The voltage and current applied to the electrodes may be externally regulated in order to exercise control over the concentration of metal ions that may be dissolved in the waste suspension 466.

Multiple sets of electrodes comprising one or more metal compositions, and having an appropriate voltage division and current flow may introduce various concentrations of one or more metal ions into the waste suspension 466.

The dissolved metal ions may act within the waste suspension 466 to deactivate or destroy bacterial, protist, fungal, and viral infectious agents present within the waste suspension 466. The deactivation or destruction of infectious agents according to embodiments of the disclosure may be herein referred to as "disinfection." Particularly suitable metal ions for use as disinfection agents include copper and silver ions. It is believed that a concentration of copper ions that is much greater than a concentration of silver ions is particularly suitable for disinfection of waste liquids. Although, a concentration ratio of 10:1 Cu to Ag has been found to be highly effective; other ratios may prove to be suitable for this application. Accordingly, ions of different metals may be produced at different concentration levels in order to provide a suitable total dissolved metal ion concentralion. In one embodiment, a suitable copper ion concentration may range from about 100 ppm to about 1000 ppm, with a further suitable example being about 400 ppm of copper ions. Likewise, suitable silver ion concentration may range from about 10 ppm to about 100 ppm, with a further suitable example being about 40 ppm of silver ions. A suitable total metal ion concentration for disinfection may range from about 110 ppm to about 1100 ppm. As a further example, a suitable total metal ion concentration may range from about 200 ppm to about 800 ppm, and as another suitable example a total metal ion concentration may range from about 300 ppm to about 600 ppm.

Accordingly, ions of different metals may be produced at different concentration levels in order to provide a suitable total dissolved metal ion concentration. A metal ion exposure time ranging from about 60 seconds to about 30 minutes may be suitable to provide disinfection to the waste stream.

The electrodes used to produce the metal ions may be pure metals in which case multiple electrodes are used and voltages and currents to each electrode are regulated in order to control the various metal ion concentrations. The electrodes may also be composed of a mixture of more than one metal, such as a metal alloy, in order to control the concentration of each ion in solution. For example, a process for producing a higher concentration of copper ions and a lower concentration of silver ions may use electrodes containing substantially more copper than silver.

In a further embodiment, the electrodes may be fabricated employing powder metallurgy. A further embodiment using copper powder and silver or silver-alloy "solder" may be employed as a binder. The powdered metal electrodes may be fabricated such that the concentration of exposed metals such as copper or silver is controlled to produce the desired concentration ratio of metal ions. Additionally, the composition of an electrode and its corresponding ionic contribution may be controlled through particle size and amount of each phase, primary metal and "binder" present in the powder molded electrode. For example, large spherical grains of copper may be pressed with silver solder powder and sintered to form an electrode with higher surface concentrations of copper.

In a further embodiment of the disclosure, the electrodes may be integrated into a mixing pump in which the vanes or other metal portions of the pump may act as electrodes.

The metal ion infusion chamber 414 may also be equipped with an inlet port 472 for flow of fluid from the receiving chamber 412 into the metal ion infusion chamber 414, and a first exit port 474 for flow of ion treated material 476 out of the metal ion infusion chamber 414. A second exit port 478 may be provided for the metal ion infusion chamber 414 for recirculation of a portion 480 of the waste material suspension 466 back into the waste stream 464 entering the meal ion infusion chamber 414. The exits ports 474 and 478 may include directionally restrictive fluid flow valves 482 and 484 that provide unidirectional fluid flow through the valves 482 and 484. A recirculation pump 486 may be included to circulate the portion 480 of the metal ion waste material suspension 466 back into the metal ion infusion chamber 414.

### Oxidation Chamber

As shown in FIG. 8, the metal ion treated material 476 may then flow into the oxidation chamber 416 for wet oxidation of the waste material. A pump 488 (FIG. 7) may be used to pump the metal ion treated material 476 through the heat exchanger or other heating means 422 and through the pressurizing pump 424 to provide a pressurized waste material 490 into the spray mixing nozzle 426 and into the chamber 416. The metal ion treated material 476 is typically preheated to a temperature ranging from about 100° C. to about 200° C. prior to introducing the material 476 to the oxidation chamber 416.

The oxygen supply 428 provides pure oxygen, or an oxygen containing gas, such as air, through a pressure regulating valve 492 and the oxygen heat exchanger or other heating means 430 into the spray mixing nozzle 426 for intimate mixing with a relatively fine mist of the material 490. The heat exchanger or other heating means 430 may be used to heat the oxygen to a temperature ranging from about 100° C. to about 350° C. before mixing the heated oxygen with the pressurized waste material 490. The oxidation chamber may be further pressurized by the compressor 432 to provide a chamber operating pressure in the range of from about 0.01 to about 0.2 MPa above ambient pressure. Removal of metal ions from the pressurized waste material 490 prior to flow of the waste material 490 into the oxidation chamber 416 is not necessary as the metal ions may further aid in the oxidation treatment step of the process.

In the oxidation chamber 416, the chemical oxygen demand (COD) and/or biological oxygen demand (BOD) of oxidized liquid 494 formed in the oxidation chamber 416 may be monitored with sensors, such as an oxygen demand sensor 495 (FIG. 8), to determine the amount of oxygen required to treat all of the incoming waste material 490. A target resulting COD or BOD for the treated waste material 498 is in a range defined by regulatory requirements at a user's location.

According to the disclosure, oxidation of the pressurized waste 490 takes place in an aqueous environment wherein water is an integral part of the reaction. Water provides a medium for dissolved oxygen to react with organics and other oxidizable materials in the waste 490. It is believed that wet oxidation involves free radical formation with oxygen derived radicals attacking the organic compounds and resulting in the formation of organic radicals.

A noteworthy characteristic of wet oxidation chemistry is the formation of carboxylic acids in addition to CO₂ and water. Other oxidation products as a result of treating the waste material 490 in the oxidation chamber, may include, but are not limited to sulfur dioxide, nitrogen dioxide, and phosphorus pentoxide which may be dissolved in the oxidized liquid 494. The yield of carboxylic acids varies greatly depending on the design of the system and may formed with about 5 to about 10 weight percent of the total organic carbon (TOC) in the waste 490. The primary carboxylic acids formed as a result of wet oxidation include acetic acid, formic acid, and oxalic acid. Such carboxylic acids are typically biodegradable and conventional biological post treatment of the oxidized liquid 494 may be conducted to reduce the amount of acids in the liquid 494.

Additional water 496 or other aqueous fluid such as a saline solution may be added to the oxidized liquid 494 in order to provide a flowable waste exit stream 498. A flow control valve 500 may be included on the exit stream 498 to maintain a suitable liquid level in the oxidation chamber 416.

The system 310 may also include a programmable microcontroller 504 capable of interfacing with automatic controllers, temperature sensors, oxygen sensors, level sensors, conductivity sensors, pH sensors, and COD and/or BOD sensors to coordinate the activities of valves, pumps, heat exchangers or other heating means, pressure regulators, and macerators, to estimate the amount of waste being processed, and to control the electrode voltage and currents responsible for producing the metal ion disinfecting agents. An ability to reverse the polarity of the electrodes may be desirable to prevent and remove a build-up of residual mineral scale on the electrodes which may impede ion generation. Additional treatment chambers may be included with the system 10 to further treat the waste stream 440 and/or 498 before discharge to a sewer system or to the environment.

The system 310 may be particularly adapted to treating waste liquid streams 440 containing consumer and industrial waste materials. Such materials may include, but are not limited to, dairy shed waste, fowl waste, milk processing plant waste, food processing wastes, waste from the wine and brewery industries, food waste, shipboard waste, petroleum wastes, wool-scouring waste, sewage, medical waste, waster paper and paper products, paper production waste, rubber waste, saw dust and wood processing waste, plastic waste, and the like. Particularly suitable waste materials include those containing bacteria, surgical waste, biological or biologically toxic materials, pharmaceutical and personal care products.

In another alternative, a macerator or mixer 502 (FIG. 7) may be included in the metal ion infusion chamber 414 for further reducing the size of any solids present in the waste material suspension 466 or for providing intimate contact between the metal ions and the waste material suspension 466.

As used throughout the specification and claims, "a" and/or "an" may refer to one or more than one. Unless otherwise indicated, all numbers expressing quantities of ingredients, properties such as molecular weight, percent, ratio, reaction conditions, and so forth used in the specification and claims are to be understood as being modified in all instances by the term "about." Accordingly, unless indicated to the contrary, the numerical parameters set forth in the specification and claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

Other embodiments of the present disclosure will be apparent to those skilled in the art from consideration of the specification and practice of the embodiments disclosed herein. Accordingly, the embodiments are not intended to be limited to the specific exemplifications set forth hereinabove. Rather, the foregoing embodiments are within the scope of the appended claims, including the equivalents thereof available as a matter of law.

The patentees do not intend to dedicate any disclosed embodiments to the public, and to the extent any disclosed modifications or alterations may not literally fall within the scope of the claims, they are considered to be part hereof under the doctrine of equivalents.

## Claims

1. A waste system (100, 310) for treating a substantially liquid waste stream (112, 440), comprising:
a metal ion generation chamber (134, 414) containing at least one metal ion generating electrode selected from one or more of copper, aluminum, zinc, bismuth, gold, and silver for generating metal ions *in situ* for disinfection of the waste stream (112, 440); and
an oxidant generation chamber (166, 416) containing one or more oxidizing agents in fluid flow communication with the metal ion generation chamber (134, 414) for denaturing the waste stream (112, 440).

2. The waste treatment system (100) of claim 1, wherein the oxidant generation chamber (166) further comprises at least one oxidant generating electrode (168, 170) for *in situ* generation of the one or more oxidizing agents, and wherein the oxidant generating electrode (168, 170) comprises one or more materials selected from the group consisting of carbon, titanium, stainless steel, and relatively inert materials.

3. The waste treatment system (100, 310) of claim 1, further comprising a maceration device (124, 418) for providing reduced particle size of material in the waste stream (112, 440).

4. The waste treatment system (100) of claim 1, further comprising a chelation chamber (194) containing a chelating agent in fluid flow communication with the oxidant generation chamber (166) for deactivating metal ions and the oxidants in the waste stream (112).

5. The waste treatment system of claim 4, wherein two or more of the metal ion generation chamber (134, 414), the oxidant generation chamber (166, 416), and the chelation chamber (194) comprise a single combined means for treating the waste stream (112, 440).

6. A method of treating a waste stream comprising:
flowing a waste stream into a waste treatment apparatus (12, 312);
generating (16, 316) metal ions selected from one or more of copper, aluminum, zinc, bismuth, gold, and silver in the waste treatment apparatus for contact with the waste stream to disinfect the waste stream; and
oxidizing (18, 318) the waste stream with one or more oxidizing agents in the waste treatment apparatus to eliminate any pharmaceutical compounds and biological activity in the waste stream.

7. The method of claim 6, wherein the one or more oxidizing agents are selected from the group consisting of oxygen, hypochlorites, peroxides, ozone, chloride ions, and chlorine radicals.

8. The method of claim 6, further comprising chelating (20) the waste stream with a chelating agent in the waste treatment apparatus to deactivate any metal ions and oxidizing chemicals remaining in the waste stream.

9. The method of claim 6, wherein the chelating agent is selected from the group consisting of EDTA, citric acid, sodium citrate, acetylacetone, ethylenediamine, diethylenetriamine, tetramethylethylenediamine, 1,2-ethanediol, 2,3-dimercaptopropanol, porphyrin, and gluconic acid.

10. The method of claim 6, further comprising macerating (14, 314) the waste stream to reduce a particles size of material in the waste stream to form a substantially liquid flowable waste stream.

## Patentansprüche

1. Abfallsystem (100, 310) zum Behandeln eines im Wesentlichen flüssigen Abfallstroms (112, 440), umfassend:
eine Metall-lon-Erzeugungs-Kammer (134, 414), welche mindestens eine Metall-Ion erzeugende Elektrode, ausgewählt aus einem oder mehreren von Kupfer, Aluminium, Zink, Wismut, Gold und Silber, zum Erzeugen von Metall-Ionen *in situ* zur Desinfektion des Abfallstroms (112, 440) enthält; und
eine Oxidans-Erzeugungs-Kammer (166, 416), welche eines oder mehrere Oxidationsmittel enthält, in Fluidströmungsverbindung mit der Metall-lon-Erzeugungs-Kammer (134, 414), zum Denaturieren des Abfallstroms (112,440).

2. Abfallbehandlungssystem (100) nach Anspruch 1, wobei die Oxidans-Erzeugungs-Kammer (166) weiterhin mindestens eine Oxidans erzeugende Elektrode (168, 170) zur *in situ* Erzeugung des einen oder der mehreren Oxidationsmittel umfasst, und wobei die Oxidans erzeugende Elektrode (168, 170) eines oder mehre Materialien, ausgewählt aus der Gruppe, bestehend aus Kohlenstoff, Titan, rostfreiem Stahl und relativ inerten Materialien, umfasst.

3. Abfallbehandlungssystem (100, 310) nach Anspruch 1, weiterhin umfassend eine Zerkleinerungsvorrichtung (124, 418; engl.: maceration device) zum Bereitstellen verringerter Partikelgröße von Material in dem Abfallstrom (112, 440).

4. Abfallbehandlungssystem (100) nach Anspruch 1, weiterhin umfassend eine Chelatisierungskammer (194), welche ein chelatisierendes Mittel enthält, in Fluidströmungsverbindung mit der Oxidans-Erzeugungs-Kammer (166), zum Deaktivieren von Metall-Ionen und der Oxidantien in dem Abfallstrom (112).

5. Abfallbehandlungssystem nach Anspruch 4, wobei zwei oder mehr der Metall-lon-Erzeugungs-Kammer (134, 414), der Oxidans-Erzeugungs-Kammer (166, 416) und der Chelatisierungskammer (194) ein einzelnes vereinigtes Mittel zum Behandeln des Abfallstroms (112, 440) umfassen.

6. Verfahren zum Behandeln eines Abfallstroms, umfassend:
Fließenlassen eines Abfallstroms in eine Abfallbearbeitungsvorrichtung (12, 312) hinein;
Erzeugen (16, 316) von Metall-Ionen, ausgewählt aus einem oder mehreren von Kupfer, Aluminium, Zink, Wismut, Gold und Silber, in der Abfallbearbeitungsvorrichtung zum Kontakt mit dem Abfallstrom, um den Abfallstrom zu desinfizieren; und
Oxidieren (18, 318) des Abfallstroms mit einem oder mehreren Oxidationsmitteln in derAbfallbearbeitungsvorrichtung, um jegliche pharmazeutische Verbindungen und biologische Aktivität in dem Abfallstrom zu eliminieren.

7. Verfahren nach Anspruch 6, wobei das eine oder die mehreren Oxidationsmittel ausgewählt sind aus der Gruppe, bestehend aus Sauerstoff, Hypochloriten, Peroxiden, Ozon, Chlorid-lonen und Chlor-Radikalen.

8. Verfahren nach Anspruch 6, weiterhin umfassend Chelatisieren (20) des Abfallstroms mit einem chelatisierenden Mittel in der Abfallbearbeitungsvorrichtung, um jegliche Metall-Ionen und oxidierende Chemikalien zu deaktivieren, welche in dem Abfallstrom verbleiben.

9. Verfahren nach Anspruch 6, wobei das chelatisierende Mittel ausgewählt ist aus der Gruppe, bestehend aus EDTA, Zitronensäure, Natriumcitrat, Acetylaceton, Ethylendiamin, Diethylentriamin, Tetramethylethylendiamin, 1,2-Ethandiol, 2,3-Dimercaptopropanol, Porphyrin und Gluconsäure.

10. Verfahren nach Anspruch 6, weiterhin umfassend Zerkleinern (14, 314; engl.: macerating) des Abfallstroms, um eine Partikelgröße von Material in dem Abfallstrom zu verringern, um einen im Wesentlichen flüssigen fließfähigen Abfallstrom zu bilden.

## Revendications

1. Système de traitement des déchets (100, 310) pour le traitement d'un courant résiduel substantiellement liquide (112, 440), comprenant :
une chambre de génération d'ions métalliques (134, 414) contenant au moins une électrode de génération d'ions métalliques consistant en un ou plusieurs éléments choisis entre le cuivre, l'aluminium, le zinc, le bismuth, le chlore et l'argent pour engendrer des ions métalliques in *situ* à des fins de désinfection du courant résiduel (112, 440) ; et
une chambre de génération d'oxydant (166, 416) contenant un ou plusieurs agents oxydants en communication par écoulement de fluide avec la chambre de génération d'ions métalliques (134, 414) pour dénaturer le courant résiduel (112, 440).

2. Système de traitement des déchets (100) suivant la revendication 1, dans lequel la chambre de génération d'oxydant (166) comprend en outre au moins une électrode de génération d'oxydant (168, 170) pour engendrer *in situ* le ou les agents oxydants, et dans lequel l'électrode de génération d'oxydant (168, 170) comprend une ou plusieurs matières choisies dans le groupe consistant en le carbone, le titane, l'acier inoxydable et des matières relativement inertes.

3. Système de traitement des déchets (100, 310) suivant la revendication 1, comprenant en outre un dispositif de macération (124, 418) pour parvenir à un diamètre réduit de particules de la matière dans le courant résiduel (112, 440).

4. Système de traitement des déchets (100) suivant la revendication 1, comprenant en outre une chambre de chélation (194) contenant un agent chélatant en communication par écoulement de fluide avec la chambre de génération d'oxydant (166) pour la désactivation des ions métalliques et des oxydants dans le courant résiduel (112).

5. Système de traitement des déchets suivant la revendication 4, dans lequel deux ou plus de deux de la chambre de génération d'ions métalliques (134, 414), de la chambre de génération d'oxydant (166, 416) et de la chambre de chélation (194) comprennent un moyen combiné unique pour le traitement du courant résiduel (112, 440).

6. Procédé pour le traitement d'un courant résiduel, comprenant :
l'écoulement d'un courant résiduel dans un appareil de traitement des déchets (12, 312) ;
la génération (16, 316) d'ions métalliques consistant en un ou plusieurs ions métalliques choisis entre le cuivre, l'aluminium, le zinc, le bismuth, le chlore et l'argent dans l'appareil de traitement des déchets pour un contact avec le courant résiduel à des fins de désinfection du courant résiduel ; et
l'oxydation (18, 318) du courant résiduel avec un ou plusieurs agents oxydants dans l'appareil de traitement des déchets pour éliminer tous composés pharmaceutiques et toute activité biologique dans le courant résiduel.

7. Procédé suivant la revendication 6, dans lequel le ou les agents oxydants sont choisis dans le groupe consistant en l'oxygène, des hypochlorites, des peroxydes, l'ozone, des ions chlorure et des radicaux chlore.

8. Procédé suivant la revendication 6, comprenant en outre la chélation (20) du courant résiduel avec un agent chélatant dans l'appareil de traitement des déchets pour désactiver tous ions métalliques et tous agents chimiques oxydants restant dans le courant résiduel.

9. Procédé suivant la revendication 6, dans lequel l'agent chélatant est choisi dans le groupe consistant en l'EDTA, l'acide citrique, le citrate de sodium, l'acétylacétone, l'éthylènediamine, la diéthylènetriamine, la tétraméthyléthylènediamine, le 1,2-éthanediol, le 2,3-dimercaptopropanol, la porphyrine et l'acide gluconique.

10. Procédé suivant la revendication 6, comprenant en outre la macération (14, 314) du courant résiduel pour réduire le diamètre de particules de la matière dans le courant résiduel afin de former un courant résiduel substantiellement liquide, apte à l'écoulement.
